# EUROPEAN PATENT SPECIFICATION

(11) **EP 1 572 952 B1**
(45) Date of publication and mention of the grant of the patent: **29.09.2010**
(21) Application number: 03762257.8
(22) Date of filing: 30.06.2003
(51) Int. Cl.: C12Q 1/68, A01N 43/04, C07H 21/04, A61K 31/07

(54) **NOVEL POLYNUCLEOTIDE AND POLYPEPTIDE SEQUENCES AND USES THEREOF**
NEUE POLYNUKLEOTID- UND POLYPEPTIDSEQUENZEN UND DEREN VERWENDUNGEN
NOUVELLES SEQUENCES POLYNUCLEOTIDIQUES ET POLYPEPTIDIQUES ET LEURS UTILISATIONS

(30) Priority: 28.06.2002 US 392675 P
(43) Date of publication of application: 14.09.2005
(60) Divisional application: 10150784.6
(73) Proprietor: Imclone LLC, New York, NY 10014 (US)
(72) Inventor: PEREIRA, Daniel, Bloomfield, NJ 07003 (US)
(74) Representative: Ingham, Stephen H.
(86) International application number: PCT/US2003/020601
(87) International publication number: WO 2004/003165

(56) References cited:
- WO-A-2004/055055
- WO-A2-01/53455
- WO-A2-01/57190
- US-B2- 6 943 241
- DATABASE EMBL [Online] 5 October 2000 (2000-10-05), "Human ORFX ORF2875 polypeptide sequence 5750" XP002442645 retrieved from EBI Database accession no. AAB43111
- DATABASE EMBL [Online] 5 October 2000 (2000-10-05), "Human ORFX ORF2875 polynucleotide sequence Seq ID 5749" XP002442646 retrieved from EBI Database accession no. AAC77320
- DATABASE GENESEQ [Online] 26 July 2001 (2001-07-26), "Human protein sequence Seq ID NO:1154" XP002442647 retrieved from EBI Database accession no. AAM25639
- KUJA-PANULA J ET AL: "AMIGO, a transmembrane protein implicated in axon tract development, defines a novel protein family with leucine-rich repeats" THE JOURNAL OF CELL BIOLOGY, ROCKEFELLER UNIVERSITY PRESS, US, vol. 160, no. 6, 17 March 2003 (2003-03-17), pages 963-973, XP003011304 ISSN: 0021-9525 -& DATABASE EMBL [Online] 19 March 2003 (2003-03-19), "Mus musculus transmembrane protein Amigo 2 mRNA" XP002442648 retrieved from EBI Database accession no. AY237006 -& DATABASE EMBL [Online] 19 March 2003 (2003-03-19), "Homo sapiens transmembrane protein AMIGO2 mRNA" XP002442649 retrieved from EBI Database accession no. AY237005
- HIPPO YOSHITAKA ET AL: "Global gene expression analysis of gastric cancer by oligonucleotide microarrays" CANCER RESEARCH, AMERICAN ASSOCIATION FOR CANCER RESEARCH, BALTIMORE, MD, US, vol. 62, no. 1, 1 January 2002 (2002-01-01), pages 233-240, XP002254861 ISSN: 0008-5472
- MORI M ET AL: "Analysis of the gene-expression profile regarding the progression of human gastric carcinoma" SURGERY, C.V. MOSBY CO., ST. LOUIS,, US, vol. 131, no. 1 Supplement, January 2002 (2002-01), pages S39-S47, XP008022195 ISSN: 0039-6060
- RABENAU K.E. ET AL: 'DEGA/AMIGO-2, a leucine-rich repeat family member, differentially expressed in human gastric adenocarcinoma: effects on ploidy, chromosomal stability, cell adhesion/migration and tumorigenicity' ONCOGENE vol. 23, no. 29, 24 June 2004, pages 5056 - 5067, XP002995776
- STRAUSBERG R.L. ET AL: 'Generation and initial analysis of more than 15,000 full-length human and mouse cDNA sequences' PROC. NATL. ACAD. SCI. vol. 99, no. 26, 24 December 2002, pages 16899 - 16903, XP002353426
- CARNINCI P. ET AL: 'High-efficiency full-length cDNA cloning' METHODS IN ENZYMOLOGY vol. 303, 1999, pages 19 - 44, XP001022979

## Description

### FIELD OF THE INVENTION

The present invention relates to polynucleotides and polypeptides that are differentially expressed in a diseased cell and methods of diagnosis, evaluation, treatment, and prevention of diseases relating thereto.

### BACKGROUND OF THE INVENTION

Cancer is the second leading cause of death in the United States, exceeded only by heart disease. In 2002, the American Cancer Society estimated that. 1,284,900 new cases will be diagnosed and 550,000 of the 1.6 million living with cancer are expected to die. Cancer is caused by genetic alteration or malfunction of the genes in the cell that results in uncontrolled proliferation of the abnormal cells to form a tumor mass or neoplasm. One of the major factors contributing to uncontrolled proliferation of cells is the over-expression of proto-oncogenes and/or under-expression of tumor suppressor genes. Both these genes play a pivotal role in cancer pathogenesis and the imbalance in polynucleotide expression of these two polynucleotide families perpetuates cancer immortalization. Classes of oncogenes and tumor suppressor genes and polypeptides include growth factors, such as epidermal growth factor (EGF) and transforming growth factor α (TNF-α) and their corresponding receptors, intracellular signaling polypeptides (tyrosine kinases), polynucleotide transcription factors, cell-cycle control polypeptides, and cell-cell or cell-matrix interacting polypeptides.

Uncontrolled cell growth leads to the formation of a localized tumor cell mass, the viability of which is maintained by the formation of neo-vasculature such as blood capillaries that are essential for ferrying nutrients to the cells. Angiogenesis is therefore a key element in the survival, growth, and metastasis of tumors. Polypeptides that are known to play a vital role in angiogenesis are growth factors and their corresponding receptors, including EGF and TGF-α, as well as vascular endothelial growth factor (VEGF).

The localized tumor mass, with its continued rapid cell proliferation, ultimately results in invasion of adjacent tissues as the tumor cells break away from the original tumor mass and migrate to regional lymph nodes and distal vital structures in the body via blood or lymphatic systems. The invasion of tumor cells requires adhesion of cells to the extra-cellular matrix, followed by its breakdown, and finally migration of the tumor cell. The dissemination of certain cancers to preferred sites have been shown to be determined by the presence of certain polypeptides on the cell surface that are capable of homing in on selected cell surface markers on target cells.

Thus, inhibition of uncontrolled cell proliferation, angiogenesis, and cell spread are some of the key targets in the development of therapeutics for managing tumor progression and recurrence. Although much progress has been made in the field of cancer and cancer therapy, many of the therapies in development today has been less than satisfactory, and a need still exists for alternative adjuvant therapy for many of these specific tumor types. The present application involves the discovery of a novel polynucleotide encoding a polypeptide or polypeptide that is differentially expressed in certain tumor cells and can be useful in the diagnosis, evaluation, treatment, and prevention of cancer.

### SUMMARY OF THE INVENTION

The present invention provides an isolated antisense oligonucleotide, wherein said oligonucleotide inhibits polynucleotide expression of SEQ ID NO: 1 or SEQ ID NO:3, wherein the oligonucleotide consists of 20-30 or 12-25 nucleotides of the coding region of SEQ ID NO: 1 or SEQ ID NO:3 or the complement thereof.
Isolated and/or purified human and mouse S30-21616/DEGA polynucleotides (hS30-21616/DEGA or mS30-21616/DEGA respectively), particularly SEQ ID NO: 1 and SEQ ID NO: 3, or a fragment thereof are provided. Isolated and/or purified human and mouse S30- 21616/DEGA polypeptides (hS30-21616/DEGA and mS30-21616/DEGA), particularly SEQ ID NO: 2 and SEQ ID NO: 4, or a fragment thereof are described. In addition, methods and compositions for diagnosis, evaluation, treatment, and prevention of diseases using such polynucleotides are provided.

### BRIEF DESCRIPTION OF THE DRAWINGS

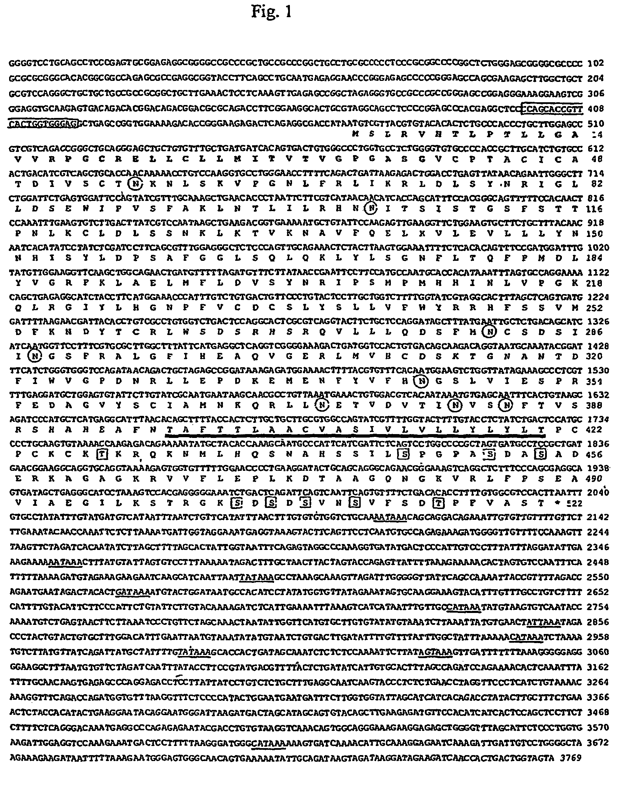

Figure 1 depicts the nucleotide and deduced amino acid sequence of S30-21616/DEGA (cDNA = 3769 bp; ORF = 1569 bp, which encodes a polypeptide of 522 amino acids) with the following features: signal peptide sequence (bold); transmembrane domain (bold underline); putative glycosylation sites (circled amino acids) and putative phosphorylated serines and threonines (boxed amino acids)

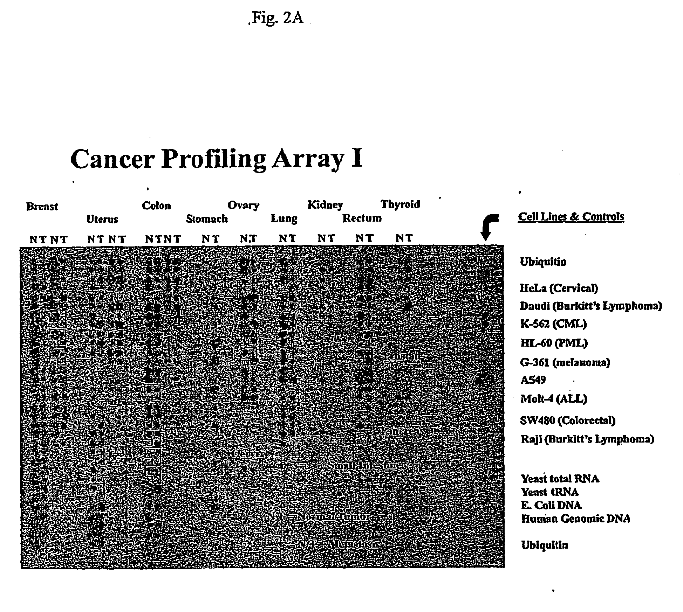

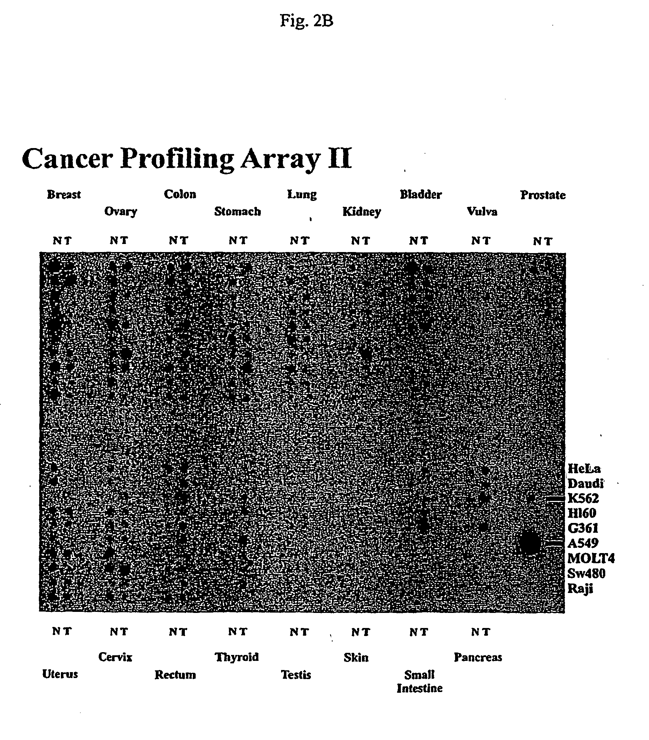

Figure 2 A and B show the dot blot analyses of S30-21616/DEGA cDNA expression levels in tumor and normal tissue samples of individual patients using BD Biosciences Cancer Profiling Arrays I (A) and II (B).

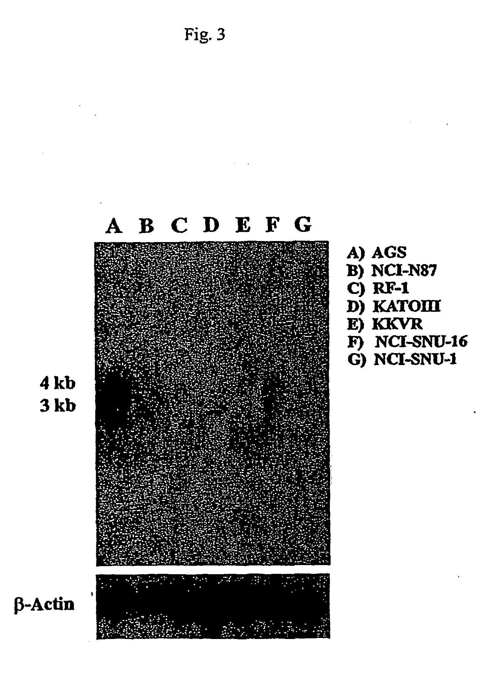

Figure 3 shows the Northern blot analysis of S30-21616/DEGA expression in gastric adenocarcinoma cell lines, AGS (Lane A), NCI-N87 (Lane B), RF-1 (Lane C), KatoIII (Lane D), KKVR (Lane E), NCI-SNU-16 (Lane F), and NCI-SNU-1 (Lane G). The bottom panel shows the mRNA level of β-actin in the respective cell lines.

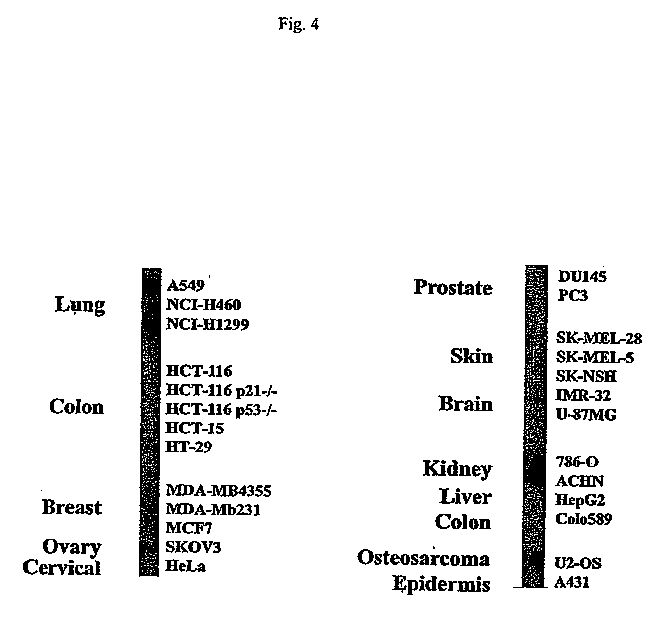

Figure 4 shows the dot blot analysis of S30-21616/DEGA expression in general, non-gastric adenocarcinoma cell lines.

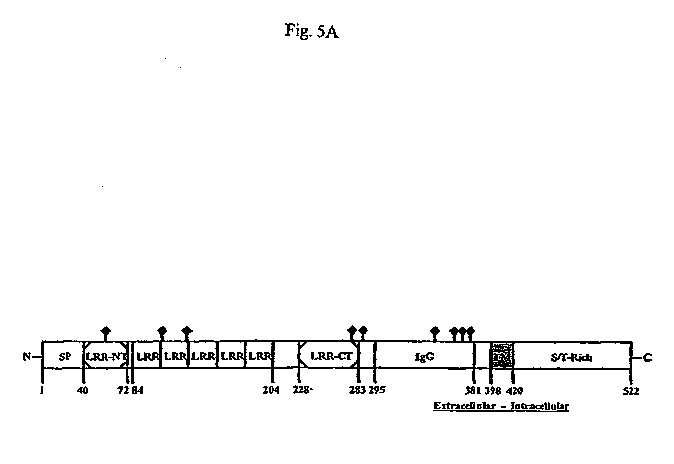

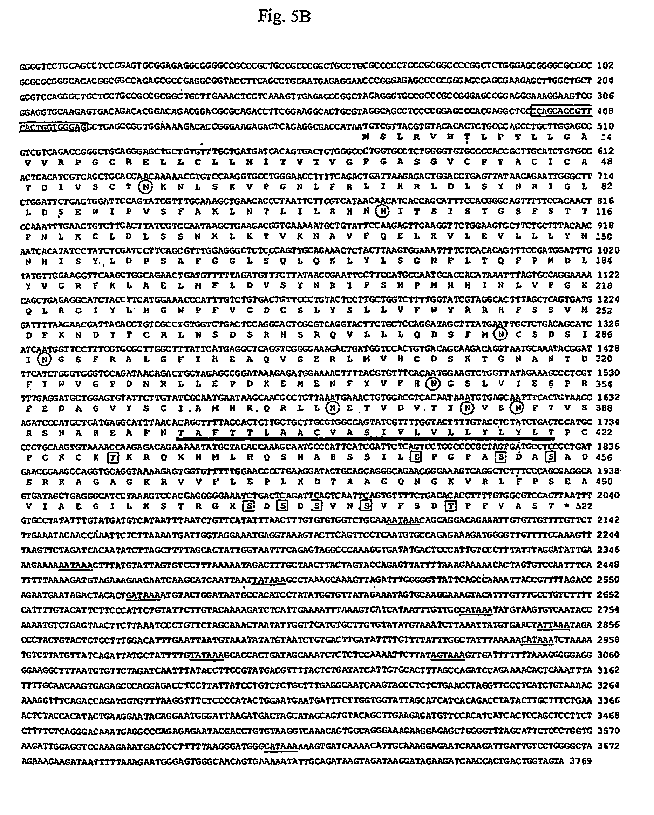

Figure 5A shows the schematic representation the S30-21616/DEGA protein. Domains present in the S30-21616/DEGA protein are shown with their amino acid boundaries as follow: SP=Signal Peptide; LRR=Leucine-Rich Repeat; LRR-NT=Cysteine-rich domain N-terminally flanking LRR; LRR-CT=Cysteine-rich domain C-terminally flanking LRR; IgG=Inmunoglobulin domain; TM=transmembrane domain; and S/T-Rich=Serine/Threonine-Rich domain. Black diamonds represent putative N-linked glycosylation sites. Figure 5B shows the amino acid sequence of the S30-21616/DEGA polynucleotide with the LRR bolded, the immunoglobulin-like domain underlined, the transmembrane domain boxed, and the casein kinase phosphorylation and polypeptide kinase C phosphorylation sites indicated by [] and (), respectively.

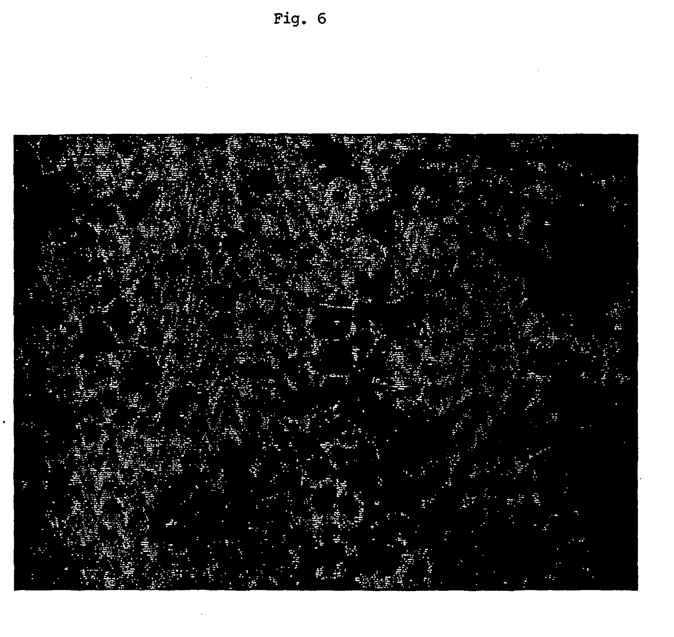

Figure 6 illustrates the sub-cellular localization of the S30-21616/DEGA- EGFP (Enhanced Green Fluorescent Protein) fusion protein that was stably expressed in 293 cells and assessed using fluorescence microscopy (200X magnification).

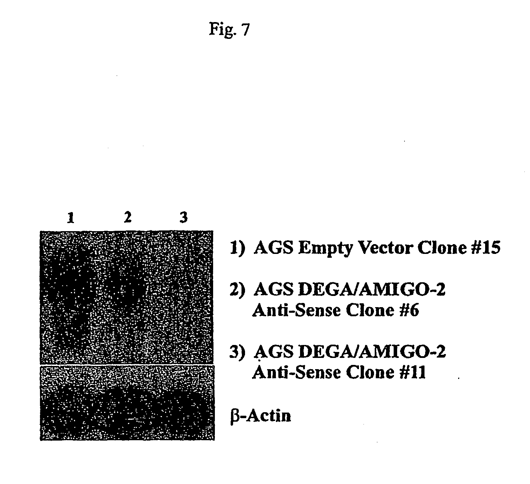

Figure 7 shows the Northern blot analysis S30-21616/DEGA expression in AGS parental or wild type cells (Lane 1), AGS transfected with empty vector, AGS/empty vector clone #15 (Lane 2), AGS transfected with S30-21616/DEGA antisense, AGS/DEGA antisense clone #6 (Lane 3) and AGS/DEGA antisense clone #11 (Lane 4). The bottom panel shows the endogenous expression of β-actin mRNA in the respective cell types (internal control).

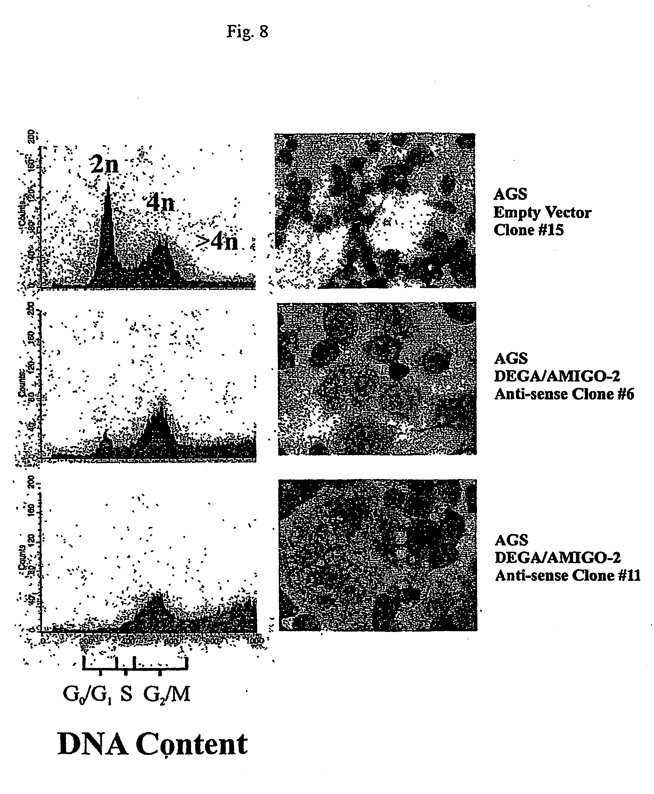

Figure 8 (left panel) shows the flow cytometric histograms of the DNA contents/cell cycle profiles of AGS empty vector clone #15 (top left panel), AGS/DEGA antisense clone #6 (middle left panel) and AGS/DEGA antisense clone #11 (bottom left panel). Figure 8 (right panel) shows the light microscopy comparing the cell size of AGS antisense clone #6 (middle right panel) and clone #11 (bottom right panel) to AGS/empty vector clone #15 (top right panel).

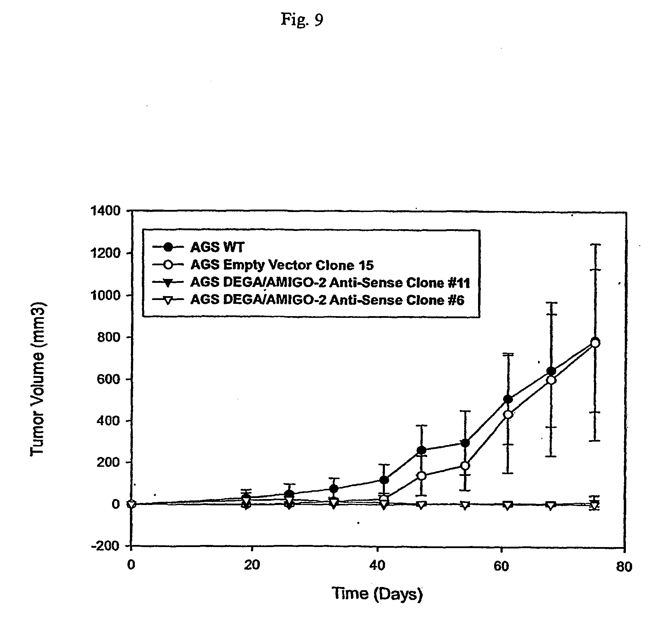

Figure 9 illustrates the tumorigenic growth curves of AGS clones stably transfected with antisense DEGA construct or empty vector determined by the number of tumor-bearing mice versus the total number of mice injected, as follow: AGS WT (12/12); AGS/empty vector clone #15 (19/19); AGS/DEGA antisense clone #6 (6/12); and AGS/DEGA antisense clone #11 (6/19).

### DETAILED DESCRIPTION OF THE INVENTION

The present invention relates to polynucleotide sequences from a lung carcinoma cell line, A549 cDNA (BD Biosciences/Clonentech, Palo Alto, CA), having a 1569 nucleotide open reading frame. This human nucleotide sequence is referred to as *hS30-21616 or hS30-21616*/*DEGA due to its Differential Expression Profile in Gastric Adenocarcinoma.* The mouse counter-part of this polynucleotide sequence, mS30-2161/DEGA, which is differentially expressed in a hematopoietic stem cell subtractive cDNA library and in a mouse kidney cancer cell line, has also been isolated. Accordingly the present invention provides an isolated antisense oligonucleotide, wherein said oligonucleotide inhibits polynucleotide expression of SEQ ID NO: 1 or SEQ ID NO: 3, wherein the oligonucleotide consists of 20-30 or 12-25 nucleotides of the coding region of SEQ ID NO: 1 or SEQ ID NO: 3 or the complement thereof.

The polynucleotides of the present invention can be DNA, RNA, DNA/RNA duplexes, polypeptide-nucleic acid (PNA), or derivatives thereof. The polynucleotide sequence includes fragments or segments that are long enough to use in polymerase chain reaction (PCR) or various hybridization techniques well known in the art for identification, cloning and amplification of all or part of mRNA or DNA molecules.
For example, hybridization under high stringency conditions means the following nucleic acid hybridization and wash conditions: hybridization at 42° C in the presence of 50% formamide ; a first wash at 65° C with 2 X SSC containing 1% SDS; followed by a second wash at 65° C with 0.1 X SSC. In addition, the polynucleotides of the present invention include complements of any of the nucleotide or peptides recited above, e. g., cDNA and mRNA.

As used herein, "isolated"or"purified"means that a molecule, e. g., a polynucleotide or polypeptide, is separated from cellular material or other components that naturally accompany it. Typically, the polynucleotide or polypeptide is substantially pure when it is at least 60% (by weight) free from the proteins and other naturally occurring organic molecules with which it is naturally associated. Preferably, the purity of the preparation is at least 75%, more preferably at least 90%, and most preferably at least 99% (by weight) free. A substantially pure polynucleotide or polypeptide can be obtained, e.g., by extraction from a natural source, expression of a recombinant nucleic acid encoding the polypeptide, or chemical synthesis. Purity can be measured by any appropriate method, e.g., column chromatography, polyacrylamide gel electrophoresis, or HPLC analysis. It should be appreciated that the term isolated or purified does not refer to a library-type preparation containing a myriad of other sequence fragments. A chemically synthesized polynucleotide or polypeptide or a recombinant polynucleotide or polypeptide produced in a cell type other than the cell type in which it naturally occurs is, by definition, substantially free from components that naturally accompany it. Accordingly, substantially pure polynucleotides or polypeptides include those having sequences derived from eukaryotic organisms but produced in E. coli or other prokaryotes.

In one embodiment of the present invention, recombinant constructs are provided. These constructs comprise a S30-21616/DEGA polynucleotide, particularly, SEQ ID NO:1 and SEQ ID NO:3, or a fragment thereof, and a suitable vector, including, but not limited to, a plasmid or viral vector, phagemid, cosmid, phage vector or derivative useful for expression, replication, probe generation and sequencing purposes. Such constructs can be used to modulate S30-21616/DEGA function.

S30-21616/DEGA function can be up-or down-regulated by modulating the transcriptional or translational control of the nucleic acid in the cell, resulting in inhibition of S30-21616/DEGA polynucleotide expression or altering the amount of S30-21616/DEGA polypeptide. By up-regulation, it is meant that the activity associated with S30-21616/DEGA is enhanced, while down-regulation means diminished or inhibition of the activity. S30-21616/DEGA polynucleotide expression control can be at the level of DNA or RNA. At the DNA level, the functions that can be inhibited includes replication or transcription. The functions of RNA to be interfered with include translocation of RNA to the site of polypeptide translation, translation of polypeptide from the RNA, splicing of RNA to yield one or more mRNA species, and catalytic activity which can be engaged in or facilitated by the RNA.

One agent or construct that can inhibit S30-21616/DEGA polynucleotide expression is an antisense oligonucleotide, which can be in the form of DNA or RNA, or a hybrid thereof. Antisense oligonucleotides can be cDNA, genomic DNA or synthetic RNA or DNA. The DNA can be double-stranded or single stranded, and if single stranded can be the coding or non-coding strand. By antisense, it is referred to specific hybridization of the oligomer with its target nucleic acid, resulting in interference with the normal function of the nucleic acid. All functions of the DNA can be interfered with by antisense, including replication and transcription. Moreover, all functions of the RNA can be interfered with by antisense, including translocation of the RNA to the site of protein translation, translation of protein from mRNA, splicing of the RNA to yield one or more mRNA species, and catalytic activity which can be engaged in or facilitated by RNA, for example. The overall effect of such antisense oligonucleotide is inhibition of expression of S30- 21616/DEGA in a cell, which may result in reduced DNA transcription, RNA translation, and polypeptide production.

Preferably, the antisense oligonucleotide has a portion of nucleotide sequence of SEQ ID NO: 1 and SEQ ID NO: 3, or the complement thereof. Also preferably, the antisense oligonucleotide is complementary to a portion of a nucleotide sequence encoding all or a portion of a S30-21616/DEGA polypeptide of SEQ ID NO: 2 and SEQ ID NO: 4. Preferably, the antisense oligonuleotide comprises short, gene- specific sequences of S30-21616/DEGA nucleic acid of 20-30 nucleotides in length and more preferably between 12-25 nucleotides in length.

The antisense oligonucleotides of the present invention can be delivered into a cell by any suitable methods, including receptor-mediated endocytosis, micro- injection and via DNA-protein complexes or cationic liposomal encapsulation. Another method of improving delivery is by attaching receptor ligands or cell specific antibodies to the antisense oligonucleotide sequences to assist in directing and guiding them to particular target cells and/or tissue regions.

One of the major problems with using antisense oligonucleotide as a therapeutic molecule is the rapid enzymatic degradation of oligonucleotides in blood and in cells. One solution is to terminally inverting the polarity of the oligonucleotide. This concept is based on the assumption that intracellular degradation is mainly due to exonuclease, and consequently, modifications at the ends of the oligonucleotides would stabilize the sequence.

Another proposed solution is via modification of the phosphodiester bonds between nucleotide bases to form, for example, phosphorothioate bonds by substituting one or more non-linking oxygen in the phosphate backbone with sulfur atoms to form, for example, monothiophosphate, and dithiophasphates. These modified oligonucleotides have been shown to have increased half lives. In addition to the sulfur modification, other types of modification in the sugar-phosphate backbone of the anti-sense oligonucleotide include, but not limited to, a 3'amino group substituted for a 3'hydroxyl group in the 2'deoxyribose ring of the DNA (phosphoramidate modification), 2'0-methyl oligo-ribonucleoside phosphodiesters, methylphosphonates (addition of a methyl group to the non-linking oxygen in the phosphodiester backbone), and phopshotriester.

A second generation of anti-sense oligonucleotide include mixed-backbone oligonucleotides which may contain monothiophosphate modification at both the 3' and 5'end of the oligonucleotide. Other examples of modifications include for example, 3'-deoxy-3'-(2-N,N-diphenyliminidazolidino)-thymidine-5'-N, N, -diisopropyl-O-methylphophoramidite, 3'-deoxy-3'-(2-N,N-diphenyliminidazolidino)-thymidine-5'-O-methylphophite. Examples of potential modifiers include for example, but not limited to hydraxine, succinate, ethylenediamine and many others. Alternatively, the 3'position of the unmodified phophosdiester bond can be substituted with a carbon in the P-O linkage to form a 3'methylene or 3'-hydroxymethylene linkage.

One of the side-effects of using antisense oligonuleotides concerns the recognition by the immune system that the antisense oligonucleotide is a foreign molecule, particularly, oligonucleotides that contain the two-base sequence, unmethylated CpG (Cytosine-phosphate-Guanine), which are commonly found in bacteria DNA and not in mammalian DNA. A potential solution is to methylate such an oligonucleotide. Other methods of minimizing the side effects of oligonucleotide treatment involves slow administration of the oligonucleotides in low doses by continuous intravenous injection (instead of administering high doses over a short period of time) and developing topical deliver vehicles that allow for sufficient delivery of the oligonucleotide via the epidermis.

It is also possible to inhibit S30-21616/DEGA expression by inactivating the nucleotide sequence encoding the S30-21616/DEGA polypeptide and hence decreasing or inhibiting polypeptide expression. Such inactivation can occur by deleting the nucleotide sequence in the cell or by introducing a deletion or mutation into the nucleotide sequence, thereby inactivating the nucleotide sequence. The nucleotide sequence can also be inactivated by inserting into the polynucleotide another DNA fragment such that expression of endogenous S30-21616/DEGA does not occur. Methods for introducing mutations, such as deletions and insertions, into genes in eukaryotic cells are well known in the art, e.g., U.S. Patent No. 5,464,764. Oligonucleotides and expression vectors useful for mutation of genes in cells can be made according to methods known in the art and guidance provided herein; for example, methods useful for making and expressing antisense oligonucleotide scan be used to make oligonucleotides and expression vectors useful for mutating genes in cells.

In another embodiment of the present invention, S30-21616/DEGA function or expression can be up-regulated or induced in a cell. Examples of up-regulation include but are not limited to administration to the cell of naked nucleic acids (naked DNA vectors such as oligonucleotides or plasmids), or polypeptides, including recombinantly produced polypeptides. Recombinantly produced polypeptides means that the DNA sequences encoding S30-21616/DEGA are placed into a suitable expression vector, which is described in detail below.

Human S30-21616/DEGA is found to be up-regulated in certain types of tumor cells, such as stomach and thyroid cancers, but generally down-regulated in breast, lung and ovarian cancers. Expression is generally low in normal or non- neoplastic tissue samples and may therefore be useful as a molecular marker for the diagnosis of various cancers. It is therefore an object of this invention that the expression or lack of expression of this polynucleotide can be used to determine the predisposition of an animal, preferably a human subject, of a specific cancer such as, but not limiting to, for example, stomach cancer. Specifically contemplated is a kit, useful in the diagnosis or prognosis of cancer, having a DNA or antisenseDNA or RNA probe derived from the sequences of the S30-21616/DEGA polynucleotide and its variants, in a mixture of reagents well know in the art.

In another embodiment of this invention, the S30-21616/DEGA polynucleotide and/or S30-21616/DEGA polypeptide can be either up-regulated and/or down-regulated. For example, up-regulation or down-regulation of the S30- 21616/DEGA on the surface of the cell or secreted into the serum of the patient with cancer can be used diagnostically to confirm or rule out suspected cancer. As a serum or cell surface biomarker, the level of expression of the polypeptide at the time of diagnosis may also provide a more precise prognosis than by staging alone. An immunoassay kit having, e.g., an antibody directed to the polypeptide or a peptide derived from the ligand binding to the polypeptide is envisioned for the present invention. The read-outs for the assays are from standard immunoassays well known in the art. Examples of such assays include, but are not limiting to, enzyme-linked immunosorbent assay (ELISA), radio-immunolabelled assay (RIA), immunoblots and fluorescein-activated cell sorting (FACs).

The invention also discloses a method to purify and produce the full length, extracellular or intracellular variants of the polypeptide. For example, one embodiment of the invention provides recombinant constructs comprising a nucleic acid of SEQ ID NO: 1 and SEQ ID NO: 3 having the polynucleotide sequence or fragment thereof, and a suitable expression vector operably linked to a promoter and a leader sequence for expression in a prokaryotic or eukaryotic host cell. Suitable prokaryotic hosts include, for example, E. coli, such as E. coli HB 101, E. coli W3110, E. coli DH1 ol; Pseudomonas, Bacillus, such as Bacillus subtilis and Streptomyces. Suitable eukaryotic host cells include yeast and other fungi, insect, and animal cells such as CHO cells, COS cells and human cells and plant cells in tissue culture. Accordingly, the present invention provides a cell transfected with an expression construct having an S30-21616/DEGA polynucleotide, e.g., SEQ ID NO: 1 and SEQ ID NO: 3, operatively linked to expression control sequences. Preferably, such a transfected cell expresses an S30-21616/DEGA polypeptide, e.g., SEQ ID NO: 2 and SEQ ID NO 4.

Any suitable neoplastic disease, i.e., cancer, can be treated using the present inventive methods, including, e.g., stomach, thyroid, breast, ovarian, kidney, or lung cancers.

The polynucleotides of the present invention can be used in the treatment of metastatic cancer. Treatments include polynucleotide ablation, polynucleotide replacement, polynucleotide expression and antisensepolynucleotide suppression. In a preferred embodiment of the invention, the method of treatment of a metastatic cancer, involves administering a pharmaceutical composition containing an effective amount of an S30-21616/DEGA polynucleotide, or fragment thereof, with additional sequences such as promoters for expression of sense and antisensemessage, recombinant sequences for targeting the gene, selectable markers for transfection and selection or replication or origins for passage in a prokaryotic, eukaryotic, bacteria, insect or yeast cells. The nucleic acids used may be single or double stranded DNA, RNA or PNA. The variants of the nucleic acids may include alterations such as deletion, substitution, addition or non-conservative alterations found naturally or engineered.

It is understood that polynucleotides and fragments of the invention, including antagonist, where used in the human body for the purpose of diagnosis or treatment, will be administered in the form of a composition additionally having a pharmaceutically-acceptable carrier. Such carriers are well known to one of average skill in the art. Modes of administration include, but are not limited to, subcutaneous, intramuscular, intravenous, intraperitoneal, intradermal or mucosal routes.

Human S30-21616/DEGA polynucleotides and polypeptides of this invention are differentially expressed in hematopoietic stem cells (see Example 2) and may be useful in the regulation of hematopoiesis. Consequently, the S30-21616/DEGA polynucleotides and polypeptides of this invention, or agents directed against such polynucleotides and polypeptides, may be useful for the treatment of stem cell associated diseases, and in particular disease of hematopoiesis such as myeloid and lymphoid cell deficiencies, for example, leukemia or in supporting the growth and proliferation of erythroid progenitor stem cells. Agonist and antagonist of the polypeptide in this invention are also envisioned in treatment of stem cell associated diseases.

The S30-21616/DEGA polynucleotides of the present invention can be administered alone or in combination with other cytokines in a pharmaceutically acceptable composition for treatment of, but not limited to, various anemias or for the use in conjunction with irradiation or chemotherapy to stimulate the production of precursors or erythroid cells ; in supporting growth and proliferation of myeloid cells, such as monocytes, macrophages and megakaryocytes; and supporting growth and proliferation of hematopoietic stem cells. In another embodiment of the invention, inhibitory molecules may be used to alter hematopoietic stem cell interacting with supporting stromal cells.

Accordingly, the present invention can be used in vivo and in vitro for investigative, diagnostic, prophylactic, or treatment methods, which are well known in the art. The examples that follow further illustrate the invention, but should not be construed to limit the scope of the invention in any way. Other embodiments and uses of the invention will be obvious to those skilled in the art from consideration of the specification and practice of the invention disclosed herein. Detailed descriptions of conventional methods, such as those employed in the construction of vectors and plasmids, the insertion of genes encoding polypeptides into such vectors and plasmids, the introduction of plasmids into host cells, and the expression and determination thereof of genes and polynucleotide products, can be obtained from numerous publication, including Sambrook, J. et al., (1989) Molecular Cloning: A Laboratory Manual, 2nd ed., Cold Spring Harbor Laboratory Press.

### EXAMPLES

### Cell Cultures

Cell lines used were obtained from the American Type Culture Collection (ATCC, Manassas, VA) and media used for their propagation were purchased from Gibco, Grand Island, NY. Cell lines and their respective growth media (shown in parentheses) were as follow: AGS, a human gastric adenocarcinoma cell line., (F-12); 293, a human kidney epithelial cell line transformed with adenovirus 5 DNA, (Dulbecco's Modified Eagles Medium (DMEM) ) ; KKVR (Iscove's DMEM); RF-1, a human gastric adenocarcinoma cell line, (Leibovitz's L-15); NCI-SNU-1, NCI-SNU- 16, NCI-N87, a human liver gastric carcinoma cell line and KatoIII, a human gastric carcinoma, (RPMI). All media were supplemented with 2 mM L-Glutamine (Gibco, Grand Island, NY) and 10% Fetal Bovine Serum (FBS; Hyclone, Logan, UT).
KatoIII and KKVR, however, were grown in 20% FBS and NCI-N87 and KKVR growth media were additionally supplemented with 1.5 g/L sodium bicarbonate.

### Example 1

The present example demonstrates identification of differentially expressed novel cancer genes. Briefly, a murine hematopoietic stem cell subtractive cDNA library was utilized. Phillips et al. (2000) Science 288: 1635-1640. Approximately 7,500 ESTs from this library were placed on DNA microarrays and hybridized with cDNA prepared from a variety of murine cancer cell lines and corresponding normal tissue.

One of the murine ESTs, S30-21616/DEGA, was identified as being 7.6 fold differentially expressed in the murine renal cell line, RAG versus normal renal tissue. This EST was sequenced and its 522 bps used to blast the NCBI non-redundant sequence databases. At this time, S30-21616/DEGA showed no homology to any known murine sequences. It did, however, show approximately 85% similarity to human chromosome 12 BAC clones and to a portion of a human endometrial cancer partial cDNA clone (IMAGE: 3625286). The IMAGE: 3625286 DNA sequence was used to generate 5'RACE ORF, and 3'UTR PCR products to give a final cDNA (hS30-21616/DEGA) of 3769 bp (Figure 1). Transcription of the cDNA resulted in various size transcripts of 2.2 and 3.5 KB, implying alternative splicing.

### Example 2

The present example demonstrates expression of hS30-21616/DEGA in normal and cancer tissues. Briefly, Northern blot analyses using multiple tissue Northern (BD Biosciences/Clonetech, Palo Alto, CA) were performed to assess the expression of hS30-21616/DEGA in normal and cancer tissues. The normal tissues screened include brain, heart, skeletal muscle, colon, thymus, spleen, kidney, liver, small intestine, placenta, lung, peripheral blood leukocyte, adrenal gland, bladder, bone marrow, lymph node, prostate, spinal cord, stomach, thyroid, trachea, uterus, and tongue. In a majority of these tissues, expression of hS30-21616/DEGA was very low.

With respect to cancer cell line expression, we assessed hS30-21616/DEGA expression by Northern blot analysis in HL-60 (Promyelocytic Leukemia), HeLa (Cervical Carcinoma), K562 (Chronic Myeloid Leukemia), Molt-4 (Acute Lymphoblastic Leukemia), Raji and Daudi (Burkitt's Lymphom), SW480 (Colorectal Adenocarcinoma), G361 (Melanoma), A549 (Lung Carcinoma) cells, AGS (human gastric adenocarcinoma), NCI-SNU-1, NCI-SNU 16, and NCI-N87 (human liver gastric carcinoma).

Human S30-21616/DEGA was expressed as multiple sized transcripts of 2-3.5 KB in A549 (data not shown) as well as in AGS and NCI-SNU-16 (see, Figure 3) cells. These size transcripts are also observed in GenBank® cDNAs expressed in testis, stomach, lung and uterus. Due to its differential expression profile in gastric adenocarcinoma, S30-21616 cDNA is henceforth named S30-21616/DEGA.

### Example 3

The present example demonstrates Cancer Profiling Array (CPA) analysis of S30-21616/DEGA expression in patient samples using cDNA dot blots. Briefly, in a variety of tumor and normal tissues (CPA) I and II (BD Biosciences Clontech, Palo Alto, CA) were probed with a cDNA fragment encompassing ORF nucleotides 1540- 2045 (numbering based on Figure 1) as per manufacturer's recommendations. The probe was labeled using [α-³²P]-dCTP (PerkinElmer Life Sciences, Inc, Boston, MA) and isolated using the Prime-It® II kit (Stratagene Cloning Systems, La Jolla, CA). CPA I and II are nylon arrays containing normalized cDNA from 241 and 160 tumor and corresponding normal tissues obtained from individual cancer patients respectively. Tissues represented by CPA I include: breast, uterus, colon, stomach, ovary, lung, kidney, rectum, thyroid, cervix, small intestine, pancreas and prostate. In addition to these tissues, CPA II carries bladder, testis, and skin tissue. These arrays also contain positive and negative controls as well as cDNA isolated from nine cancer cell lines: HeLa, Daudi, K-562, HL-60, G-361, A549, Molt-4, SW480 and Raji.

The results from the array profile in Figures 2A (CPA 1) and 2B (CPA II) again showed that DEGA was expressed in some normal lung, colon and rectum samples but the expression are generally lower than in the female reproductive, tissues. Nearly every patient showed strong expression of DEGA in normal breast tissues. In contrast, DEGA expression in tumor samples was differentially expressed in 56% of thyroid (9/16; 5/6 in CPA I, and 4/10 in CPA II) and 45% of stomach cancers (17/38; 14/28 in CPA I and 3/10 in CPA II). Of the cancer cell lines tested in CPA I (Figure 2A) and CPA II (Figure 2B), A549 expressed high levels of DEGA cDNA and K562 expressing approximately 10 fold less DEGA cDNA than A549 cells. There was minimal expression in Molt-4, G361 and HeLa cells.

Table 1 further provides a summary of the regulated differential expression of DEGA from various tissues using the arrays. The expression levels were normalized against cDNA from 241 tumor and corresponding normal tissues from individual patients.

**TABLE 1**

| Type of Cancer | Number of Samples | Up Regulated | Down Regulated | No Difference |
|---|---|---|---|---|
| Stomach | 28 | 45% | 34% | 21% |
| Thyroid | 6 | 83% | 0% | 17% |
| Breast | 50 | 6% | 70% | 24% |
| Ovary | 14 | 0% | 43% | 57% |
| Lung | 21 | 19% | 57% | 24% |
| Uterus | 42 | 19% | 26% | 55% |
| Colon | 34 | 26% | 26% | 48% |
| Cervix | 1 | 0% | 0% | 100% |
| Kidney | 20 | 10% | 10% | 80% |
| Rectum | 18 | 11% | 11% | 78% |
| Prostate | 4 | 0% | 50% | 50% |
| Pancreas | 1 | 0% | 0% | 100% |
| Small Intestine | 2 | 0% | 0% | 100% |

In twenty-eight of the stomach and six thyroid cancers profiled, hS30- 21616/DEGA was up-regulated in 45% and 83% of tumor respectively versus normal samples. However, in fifty breast, fourteen ovarian, and twenty one lung cancers typed, hS30-21616/DEGA was down-regulated in 70%, 43% and 57% of tumor respectively versus normal samples. The results showed that hS30-2161 6/DEGA is differentially expressed in different tumor types and may play a role in modulating tumor growth.

### Example 4

The present example demonstrates Northern Blot analysis of DEGA expression in gastric adenocarcinoma cells. The observation that 45% of the stomach cancer patient samples showed differential expression of DEGA in their tumors but insignificant expression in adjacent normal stomach tissue suggests that DEGA may play a pivotal role in the development or progression of at least a sub-fraction of gastric adenocarcinoma. Northern Blot analysis was carried out to determine whether DEGA was expressed in the adenocarcinoma cell lines, AGS, NCI-SNU-1, NCI- SNU-16, NCI-N87, KATOIII, KKVR and RF-1.

The autoradiograph in Figure 3 shows that expression in both AGS (Lane A) and NCI-SNU-16 (Lane F) cells, with AGS having eight-fold greater expression over NCI-SNU-16.

### Example 5

The present example demonstrates Cancer Profiling Array (CPA) analysis of S30-21616/DEGA expression in other non-gastric adenocarcinoma cancer cell lines. Using cDNA dot blots, the S30-21616/DEGA probe and conditions used for CPA I and II were also used to hybridize BD Bioscience's Cancer Cell Line Profiling Array, which contains cDNA from cell lines representing cancer of the lung (A549, NCI-H- 460, NCI-H1299), colon (HCT-116, HCT-15, HT-29), breast (MDA-MB4355, MDA- MB231, MCF7), ovary (SK-OV-3), cervix (HeLa), prostate (DU-145, PC-3), skin (SK-MEL-28, SK-MEL-5, SK-NSH, IMR-32, U-87MG), brain (IMR-32, U-87MG), kidney (786-O, ACHN), liver (Hep-G2), colon (Colo589), osteosarcoma (U2-OS), and epidermis (A-431).

The results showed that the highest expression was observed in lung NCI- H1299 and A549 cells (Figure 4, left panel), kidney ACHN cells and U2-OS osteosarcoma cells (Figure 4, right panel). Lower but significant expression was observed in renal line, 786-O (Figure 4, right panel), MDA-MB-231 breast cells and ovarian cancer cell line, SKOV-3 (Figure 4, left panel).

### Example 6

The present example demonstrates S30-21616/DEGA cDNA cloning. Briefly, Human S30-21616/DEGA cDNA was amplified as a RT-PCR product derived from Marathon-Ready™ A549, a human lung cell carcinoma line and spleen cDNA preparations (BD Biosciences/Clonetech, Palo Alto, CA). The PCR primers used to amplify the polynucleotide of interest are forward PCR Primer No. 1 with the sequence 5'ATG TCG TTA CGT GTA CAC ACT CTG 3' (SEQ ID NO: 14) and reverse PCR Primer No. 2,5' TTA AGT GGA CGC CAC AAA AGG TGT G 3' (SEQ ID NO : 15) (Bio-Synthesis Incorporated, Lewisville, TX; start and stop codons are underlined). The PCR reaction was performed using standard RT-PCR protocols well known in the art, with the exception of the polymerase used, which is Titanium Taq polymerase (BD Biosciences/Clonetech, Palo Alto, CA), specifically the RT-PCR condition described in the Marathon-Ready™ cDNA manual was utilized: 94°C, 30 seconds; 68°C, 2 minutes.

The S30-21616/DEGA ORF PCR product (1569 bp) was cloned into pCR2.1 (InVitrogen Corporation, TOPO TA® Cloning Kit, Carlsbad, CA) and sequence verified (Molecular Genetics Instrumentation Facility, University of Georgia, Athens, GA; GENEWIZ Inc, North Brunswick, NJ). To obtain DNA sequence upstream of the S30-21616/DEGA start codon; 5'RACE was performed using A549 Marathon- Ready cDNA as explained above with few modifications. Asp1, a forward primer (included with the Marathon-Ready™ cDNA) and a S30-21616/DEGA gene-specific reverse primer (5'AAC TCA GGT CCA GTC TCT TAA TCA G 3' ; SEQ ID NO : 16) produced a PCR product after 35 rounds of amplification (94°C, 30s; 57°C, 30 sec; 68°C, 2 min. ) that was subcloned into pCR2.1 and sequenced verified. Sequence representing the 3'UTR of S30-21616/DEGA was initially obtained computationally from the human chromosome 12 BAC clone, GS1-99H8 (GenBank® accession #AC004010). A series of PCR primers were subsequently designed to determine the longest 3'UTR sequence present in the A549 cDNA preparation. The following primer set was used to identify the longest 3'UTR segment: forward primer 5'ATG TCG TTA CGT GTA CAC ACT CTG 3' (SEQ ID NO: 17); start codon underlined and reverse primer 5'CAA AAT GAA AAG ACA GGC AAA CAA ATG 3' (SEQ ID NO: 18), which initiates at X nucleotides 3'of the S30-2161 6/DEGA ORF.

Figure 5A schematically shows the structural features of the gene product of S30-21616/DEGA. The protein possesses a signal sequence and a transmembrane domain suggesting that it may localize to the cell surface. Its putative extracellular portion contains an Ig domain and 5 leucine-rich repeats (LRR) flanked by cysteine residues present in LRR-N-terminal (LRR-NT) and LRR-C-terminal (LRR-CT) domains. Consequently, S30-21616/DEGA appears to belong to the LRR superfamily. The putative cytosolic portion of S30-21616/DEGA contains 102 amino acids and lacks the presence of any known protein domains. Approximately 1/5 or 20 of the cytosolic residues are either a serine or a threonine, suggesting that S30- 21616/DEGA may function as a signaling molecule in the cell. Taken together, protein sequence analysis suggests that the S30-21616/DEGA gene product may function as a signaling cell adhesion molecule. The corresponding amino acid sequence of the protein is illustrated in Figure 5B.

### Example 7

The present example demonstrates stable expression of an S30-21616/DEGA- EGFP fusion construct in 293 cells and sub-cellular localization of the protein.
Briefly, a fusion protein was engineered whereby an enhanced green fluorescent protein (EGFP) was fused to its C-terminus. The S30-21616/DEGA ORF was PCR amplified and cloned into XhoI/AgeI sites of pEGFP-N1 (BD Biosciences Clontech, Palo Alto, CA). The PCR reaction was performed as described in the S30- 21616/DEGA cDNA cloning section with the exception that the forward primer used (5'ATC CTC GAG GCG ACC ATA ATG TCG TTA CGT GTA CAC ACT 3'; SEQ ID NO : 19), which contained the native Kozak sequence and a Xho I site (underlined) 5'to the start codon shown in bold. The reverse primer used (5'GAT CAC CGG TGC AGT GGA CGC CAC AAA AGG TGT GTC 31; SEQ ID NO: 20) contained an Age I site (underlined) 3'of the stop codon shown in bold.

The S30-21616/DEGA-EGFP construct was stably transfected into 293 cells using FuGENE6 transfection reagent (Roche Molecular Biochemicals, Indianapolis, IN) as outlined by the manufacturer. A 2: 1 FuGENE6 : DNA ratio was used.
Transfected cells were left for 2 days at 37°C after which time they were plated in limiting dilution and subjected to selection with 800 µg/ml Geneticin (Sigma-Aldrich Corp., St. Louis, MO). Individual clones were isolated by trypsinization using cloning rings and allowed to expand.

To determine the sub-cellular localization of the S30-21616/DEGA-EGFP fusion protein, clones were assessed using a Zeiss Axioskop fluorescent microscope (200x magnification). The fluorescent micrograph shown in Figure 7 confirmed cell surface localization of the S30-21616/DEGA protein.

### Example 8

The present example demonstrates stable expression of S30-21616/DEGA antisenseconstructs in AGS cells. The expression profile of S30-21616/DEGA suggests that it may play a functional role in the development or progression of a sub- set of human gastric adenocarcinoma. To address the functional role of S30- 21616/DEGA, human AGS cell line that has been shown to express significant levels of S30-21616/DEGA mRNA was transfected to stably express antisenseS30- 21616/DEGA construct or an empty vector. The cells lines established were used in comparison studies of clones in cell cycle, proliferation and tumorigenicity assays.

To generate S30-21616/DEGA antisenseclones, a nucleotide fragment encompassing the entire ORF of S30-21616/DEGA as well as one that comprised the 5'most 608 nucleotides of its ORF (generated from a partial Eco-RI digest of pCR2.1- S30-21616/DEGA) were cloned into pIRES PURO2 (BD Biosciences Clontech, Palo Alto, CA) in reverse orientation and stably transfected into AGS cells. Concurrently, pIRES PURO2 was also stably transfected into these cells to serve as an empty vector control. Transfection and clone isolation were performed exactly as described for the S30-21616/DEGA-EGFP fusion with the exception that cells were selected with 400, µg/ml puromycin (Sigma-Aldrich Corp. , St. Louis, MO).

### Example 9

The present example demonstrates comparative expression of AGS/DEGA antisense and empty vector clones by Northern Blot snalysis. To determine the effect of S30-21616/DEGA expression in gastric adenocarcinoma cell line, AGS stably transfected with antisenseS30-21616/DEGA and empty vector, standard Northern- blotting procedures were carried out. Total RNA was isolated using the Micro-to- Midi Total RNA system (In Vitrogen Corporation, Carlsbad, CA) exactly as recommended by the manufacturer and 5 µg was resolved on a 1.2% denaturing formaldehyde gel and transferred to Nytran® SuPerCharge membranes (Schleicher & Schuell, Inc., Keene, NH). Blots were hybridized with the ³²P random-labeled S30- 21616/DEGA C-terminal probe used to probe the CPA I and II cDNA blots. Hybridizations were performed for one hour at 68°C using ExpressHyb™ solution (BD Biosciences Clontech, Palo Alto, CA). Three 10 minute room temperature washes (2 X SSC, 0.05% SDS) and two 20 minute 50°C washes (0.1 X SSC, 0.1% SDS) followed. Membranes were rinsed in 2 X SSC and exposed overnight at-70°C to Kodak BioMax MR film. (Eastman Kodak Company, Rochester, NY.)

Northern blot analysis of AGS/DEGA antisenseclones showed significant and stable down-regulation of S30-21616/DEGA expression. Figure 8 shows a decreased in level of mRNA expression for AGS/DEGA antisenseclone #6 (lane 3) and clone #11 (lane 4) compared to untransfected or wild type (WT) AGS parental cells (lane 1) and AGS empty vector clone #15 (lane 2). The lower panel shows the mRNA expression of β-action in the cells tested as controls.

### Example 10

The present example demonstrates DNA content/cell cycle profiles of AGS/DEGA antisenseclones and empty vector clones by flow cytometry analysis. To measure DNA content and therefore cell cycle profile, flow cytometry was utilized.
AGS empty vector and S30-21616/DEGA antisenseclones were grown asynchronously in serum-containing growth media, trypsinized, washed once in PBS and stained for 10 minutes at room temperature in the dark with the propidium iodide solution provided in the DNA QC Particles kit (Catalog # 349523; Becton Dickinson). Cells were subsequently analyzed on a Becton Dickinson FACSVantage™ flow cytometer using parameters outlined in the DNA QC particles kit. Cell size was assessed by light mircroscopy.

Flow cytometric analysis of the propidium iodide stained cells showed that AGS/empty vector cells demonstrated a typical DNA content/cell cycle phase profile for proliferating cells, with a majority of cells in G₀/G₁, followed in quantity by cells in G₂/M and S phase (see, Figure 9, left panel, top histogram). In contrast AGS/DEGA antisenseclones #6 (see, Figure 9, left panel, middle histogram) and #11 (see, Figure 9, left panel, bottom histogram) displayed a dramatic alteration in the cell cycle profile. Nearly all cells displayed a shift towards the G₂/M phase of the cell cycle. Clone #11 showed slightly greater shift towards the G₂/M phase than clone #6. The shift to G₂/M also correlated to the increase in cell size observed for both AGS/DEGA antisenseclone #6 and #11 (see, Figure 9, bottom two right panel) in contrast to the cell size observed for AGS/empty vector cells (see, Figure 9, top right panel).

### Example 11

The present example demonstrates in-vivo tumorigenicity studies of DEGA-expressing cells. Given the cell cycle profile differences observed between AGS/empty vector and AGS/DEGA antisenseclones, a difference in cell proliferation *in vivo* is also expected. Since AGS cells are tumorigenic in nude mice, the effect of S30-216161DEGA antisensein suppressing S30-21616/DEGA expression and hence negatively influence the tumorigenic potential of AGS cells was carried out as follows. AGS clones were suspended in growth media, placed on ice and thoroughly mixed with MATRIGEL^{™} (Collaborative Research Biochemicals, Bedford, MA) at a 1:1 (v/v) ratio. MATRIGEL^{™} is derived from the Engelbroth-Holm-Swarm mouse sarcoma, which has been found to be a rich source of the ECM proteins: laminin, collagen IV, nidogen/enactin and proteoglycan (12). When mixed with cell lines, MATRIGEL^{™} enhances tumorigenesis (27). For each athymic (nu/nu) mouse (Charles River Laboratories, Wilmington, MA), ten million cells were injected subcutaneously and tumor volumes (mm³) were measured weekly using calipers for 11 weeks.

Figure 10 depicts the results of tumor growth study in mice. After 75 days, 12/12 AGS cell-injected mice and 19/19 AGS/empty vector clone #15 injected mice developed tumor with a mean tumor volume of approximately 800 mm³. In sharp contrast, only 6/12 mice injected with AGS/DEGA antisenseclone #6 and 6/19 mice injected with AGS/DEGA antisenseclone #11 developed tumors with a mean tumor volume of approximately 20 mm³.
**SEQ ID NO: 1**
   **hS30-21616/DEGA cDNA sequence:**
   The open reading frame is shown in upper case. Bold letters = start and stop codons. For more info on sequence, see Figure 1 of manuscript. 
ggggtcctgcagcctcccgagtgcggagaggcggggccgcccgctgccgcccggctgcc
tgcgcccccctcccgcggccccggctctgggagcggggcgccccgcgcgcgggcacacgg
cggccagagcgccgaggcggtaccttcagcctgcaatgagaggaacccgggagagcccc
cgggagccagcgaagagcttggctgctgcgtccagggctgctgctgccgccgcggctgc
ttga
aactcctcaaagttgagagccggctagagggtgccgcccgccgggagccggagggaaag
gaagtcgggaggtgcaagagtgacagacacggacagacggacgcgcagaccttcggaag
gcactgcgtaggcagcctccccggagcccacgaggctccccagcaccgttcactggtgg
gaggctgagccggtggaaaagacaccgggaagagactcagaggcgaccataATGTCGTT
ACGT
GTACACACTCTGCCCACCCTGCTTGGAGCCGTCGTCAGACCGGGCTGCAGGGAGCTGCT
GTGTTTGCTGATGATCACAGTGACTGTGGGCCCTGGTGCCTCTGGGGTGTGCCCCACCG
CTTGCATCTGTGCCACTGACATCGTCAGCTGCACCAACAAAAACCTGTCCAAGGTGCCT
GGGAACCTTTTCAGACTGATTAAGAGACTGGACCTGAGTTATAACAGAATTGGGCTTCT
GGAT
TCTGAGTGGATTCCAGTATCGTTTGCAAAGCTGAACACCCTAATTCTTCGTCATAACAA
CATCACCAGCATTTCCACGGCAGTTTTTCCACAACTCCAAATTTGAAGTGTCTTGACT
TATCGTCCAATAAGCTGAAGACGGTGAAAAATGCTGTATTCCAAGAGTTGAAGGTTCTG
GAAGTGCTTCTGCTTTACAACAATCACATATCCTATCTCGATCCTTCAGCGTTTGGAGG
GCTC
TCCCAGTTGCAGAAACTCTACTTAAGTGGAAATTTTCTCACACAGTTTCCGATGGATTT
GTATGTTGGAAGGTTCAAGCTGGCAGAACTGATGTTTTTAGATGTTTCTTATAACCGAA
TTCCTTCCATGCCAATGCACCACATAAATTTAGTGCCAGGAAAACAGCTGAGAGGCATC
TACCTTCATGGAAACCCATTTGTCTGTGACTGTTCCCTGTACTCCTTGCTGGTCTTTTG
GTAT
CGTAGGCACTTTAGCTCAGTGATGGATTTTAAGAACGATTACACCTGTCGCCTGTGGTC
TGACTCCAGGCACTCGCGTCAGGTACTTCTGCTCCAGGATAGCTTTATGAATTGCTCTG
ACAGCATCATCAATGGTTCCTTTCGTGCGCTTGGCTTTATTCATGAGGCTCAGGTCGGG
GAAAGACTGATGGTCCACTGTGACAGCAAGACAGGTAATGCAAATACGGATTTCATCTG
GGTG
GGTCCAGATAACAGACTGCTAGAGCCGGATAAAGAGATGGAAAACTTTTACGTGTTTCA
CAATGGAAGTCTGGTTATAGAAAGCCCTCGTTTTGAGGATGCTGGAGTGTATTCTTGTA
TCGCAATGAATAAGCAACGCCTGTTAAATGAAACTGTGGACGTCACAATAAATGTGAGC
AATTTCACTGTAAGCAGATCCCATGCTCATGAGGCATTTAACACAGCTTTTACCACTCT
TGCT
GCTTGCGTGGCCAGTATCGTTTTGGTACTTTTGTACCTCTATCTGACTCCATGCCCCTG
CAAGTGTAAAACCAAGAGACAGAAAAATATGCTACACCAAAGCAATGCCCATTCATCGA
TTCTCAGTCCTGGCCCCGCTAGTGATGCCTCCGCTGATGAACGGAAGGCAGGTGCAGGT
AAAAGAGTGGTGTTTTTGGAACCCCTGAAGGATACTGCAGCAGGGCAGAACGGAAAGT
CAGG
 
CTCTTTCCCAGCGAGGCAGTGATAGCTGAGGGCATCCTAAAGTCCACGAGGGGAAATC
TGACTCAGATTCAGTCAATTCAGTGTTTCTGACACACCTTTTGTGGCGTCCACTTAAt
ttgtgcctatatttgtatgatgtcataatttaatctgttcatatttaactttgtgtgtg
gtctgcaaaataaacagcaggacagaaattgtgttgttttgttctttgaaatacaacca
aatt
ctcttaaaatgattggtaggaaatgaggtaaagtacttcagttcctcaatgtgccagag
aaagatggggttgttttccaaagtttaagttctagatcacaatatcttagcttttagca
ctattggtaatttcagagtaggcccaaaggtgatatgactcccattgtccctttattta
ggatattgaaagaaaaataaactttatgtattagtgtcctttaaaaatagactttgct
aact
tactagtaccagagttattttaaagaaaaacactagtgtccaatttcatttttaaaaga
tgtagaaagaagaatcaagcatcaattaattataaagcctaaagcaaagttagatttgg
gggttattcagccaaaattaccgttttagaccagaatgaatagactacactgataaaat
gtactggataatgccacatcctatatggtgttatagaaatagtgcaaggaaagtacatt
tgtt
tgcctgtcttttcatttgtacattcttcccattctgtattcttgtacaaaagatctca
ttgaaaatttaaagtcatcataatttgttgccataaatatgtaagtgtcaataccaaaa
tgtctgagtaacttcttaaatccctgttctagcaaactaatattggttcatgtgcttgt
gtatatgtaaatcttaaattatgtgaactattaaatagaccctactgtactgtgctttg
gaca
tttgaattaatgtaaatatatgtaatctgtgacttgatattttgttttatttggctatt
taaaaacataaatctaaaatgtcttatgttatcagattatgctattttgtataaagcac
cactgatagcaaatctctctccaaaattcttatagtaaagttgatttttttaaagggg
aggggaaggctttaatgtgttctagatcaatttataccttccgtatgacgttttactct
gata
tcattgtgcactttagccagatccagaaaacactcaaatttattttgcaacaagtgaga
gcccaggagacctccttattatcctgtctctgctttgaggcaatcaagtaccctctctg
aacctaggttccctcatctgtaaaacaaaggtttcagaccagatggtgtttaaggtttc
tccccatactggaatgaatgatttcttggtggtattagcatcatcacagacctatactt
gctt
tctgaaactctaccacatactgaaggaatacaggaatgggattaagatgactagcatag
cagtgtacagcttgaagagatgttccacatcatcactccagctccttctcttttctcag
ggacaaatgaggcccagagagaatacgacctgtgtaaggtcaaacagtggcagggaaag
aaggagagctggggtttagcattctccctggtgaagattggaggtccaaagaaatgact
cctt
tttaagggatgggcataaaaaagtgatcaaaacattgcaaaggagaatcaagattgat
tgtcctggggctaagaaagaagataattttaaagaatgggagtgggcaacagtgaaaa
atattgcagataagtagataaggatagaagatcaaccactgactggtagta
**SEQ ID NO:2**
   **hS30-21616/DEGA amino acid sequence:** 
MSLRVHTLPTLLGAVVRPGCRELLCLLMITVTVGPGASGVCPTACICATDIVSCTNKNL
SKVPGNLFRLIKRLDLSYNRIGLLDSEWIPVSFAKLNTLILRHNNITSISTGSFSTTPN
LKCLDLSSNKLKTVKNAVFQELKVLEVLLLYNNHISYLDPSAFGGLSQLQKLYLSGNFL
TQFPMDLYVGRFKLAELMFLDVSYNRIPSMPMHHINLVPGKQLRGIYLHGNPFVCDCSL
YSLL
VFWYRRHFSSVMDFKNDYTCRLWSDSRHSRQVLLLQDSFMNCSDSIINGSFRALGFIHE
AQVGERLMVHCDSKTGNANTDFIWVGPDNRLLEPDKEMENFYVFHNGSLVIESPRFEDA
GVYSCIAMNKQRLLNETVDVTINVSNFTVSRSHAHEAFNTAFTTLAACVASIVLVLLYL
YLTPCPCKCKTKRQKNMLHQSNAHSSILSPGPASDASADERKAGAGKRVVFLEPLKDTA
AGQN
GKVRLFPSEAVIAEGILKSTRGKSDSDSVNSVFSDTPFVAST.
**SEQ ID NO: 3**
   **Polynucleotide sequence of mouse S30-21616, *mS30-21616*/*DEGA.*** 
ATGGTGAGCCCCAGCGCCTCAGGAATGTGCCCCACTGCTTGCATCTGTGCC
ACCGACATTGTCAGCTGCACCAACAAAAACCTATCTAAGGTGCCCGGAA
CCTTTTCAGACTGATTAAAAGACTGGATCTGAGCTATAACAGAATCGGAC
TGTTGGATGCCGACTGGATCCCGGTGTCGTTTGTCAAGCTGAGCACCTTAA
TTCTTCGCCACAACAACATCACCAGCATCTCCACGGGCAGTTTCTCCACAA
CCCCAAATTTAAAGTGTCTGGACTTATCATCCAATAGGCTGAAGTCGGTA
AAGAGTGCCACATTCCAAGAGCTGAAGGCTCTGGAAGTACTGCTGCTGTA
CAACAACCACATTTCCTATCTGGACCCCGCAGCGTTCGGGGGGCTTTCCCA
CTTGCAGAAACTCTATCTGAGTGGGAACTTTCTCACACAGTTCCCTATGGA
TTTGTATACTGGGAGGTTCAAGCTGGCTGATCTGACATTTTTAGATGTTTC
CTATAATCGAATCCCTTCCATACCGATGCACCATATAAACTTAGTGCCGGG
GAGACAGCTGAGAGGCATCTACCTTCACGGGAACCCATTTGTATGTGACT
GTTCTCTGTACTCGTTGCTGATCTTTTGGTACCGTAGGCACTTTAGCTCCGT
GATGGATTTTAAGAATGACTATACCTGTCGCCTGTGGTCTGACTCCAGGCA
CTCCCACCAGCTGCAGCTGCTCCAGGAGAGCTTTCTGAACTGTTCTTACAG
CGTTATCAACGGCTCCTTCCACGCACTTGGCTTTATCCACGAGGCTCAGGT
TGGGGAGAGGGCGATCGTCCACTGTGACAGCAAGACTGGCAATGGAAAT
ACTGATTTCATCTGGGTCGGTCCCGATAACAGGCTGCTGGAGCCAGATAA
AGACATGGGGAACTTTCGTGTGTTTACAACGGAAGTCTGGTCATAGAGA
ACCCTGGCTTTGAGGACGCCGGGGTATATTCTTGTATCGCAATGAACAGG
CAGCGGCTGTTAAACGAGACGGTGGATATCATGATCAACGTGAGCAATTT
CACCATAAACAGATCCCACGCCCACGAGGCGTTTAACACGGCCTTTACCA
CCCTGGCTGCCTGCGTGGCCAGTATAGTT
CTAGTGCTACTGTATCTGTACCTGACGCCGTGCCCATGCAAATGCAAAGCC
AAGAGACAGAAAAACACGCTGAGCCAAAGCAGTGCCCACTCGTCCATTCT
CAGTCCTGGCCCCACTGGCGATGCCTCTGCTGACGATCGGAAGGCAGGTA
AAAGAGTCGTGTTTCTGGAGCCCCTGAAGGACACGGCGGCCGGACAGAAT
GGCAAAGTCAAGCTTTTCCCCAGTGAGACCGTTATAGCCGAGGGCATCTT
AAAGTCCACCAGGGCAAAGTCTGACTCAGACTCAGTCAATTCCGTGTTCT
CAGACACACCCTTTGTGGCATCCACTTAG
**SEQ ID NO: 4**
   **Amino acid sequence of mouse S30-21616 polypeptide, mS30-21616/DEGA.** 
MVSPSASGMCPTACICATDIVSCTNKNLSKVPGNLFRLIKRLDLSYNRIGLLDA
DWIPVSFVKLSTLILRHNNITSISTGSFSTTPNLKCLDLSSNRLKSVKSATFQEL
KALEVLLLYNNHISYLDPAAFGGLSHLQKLYLSGNFLTQFPMDLYTGRFKLA
DLTFLDVSYNRIPSIPMHHINLVPGRQLRGIYLHGNPFVCDCSLYSLLIFWYRR
HFSSVMDFKNDYTCRLWSDSRHSHQLQLLQESFLNCSYSVINGSFHALGFIHE
AQVGERAIVHCDSKTGNGNTDFIWVGPDNRLLEPDKDMGNFRVFYNGSLVIE
NPGFEDAGVYSCIAMNRQRLLNETVDIMINVSNFTINRSHAHEAFNTAFTTLA
ACVASIVLVLLYLYLTPCPCKCKAKRQKNTLSQSSAHSSILSPGPTGDASADD
RKAGKRVVFLEPLKDTAAGQNGKVKLFPSETVIAEGILKSTRAKSDSDSVNSV
FSDTPFVAST
**SEQ ID NO: 5**
   **Amino acid sequence of human S30-21616/DEGA polypeptide lacking transmembrane (TM) domain, -TM hS30-21616/DEGA.** 
MSLRVHTLPTLLGAVVRPGCRELLCLLMITVTVGPGASGVCPTACICATDIVS
CTNKNLSKVPGNLFRLIKRLDLSYNRIGLLDSEWIPVSFAKLNTLILRHNNITSI
STGSFSTTPNLKCLDLSSNKLKTVKNAVFQELKVLEVLLLYNNHISYLDPSAF
GGLSQLQKLYLSGNFLTQFPMDLYVGRFKLAELMFLDVSYNRIPSMPMHHIN
LVPGKQLRGIYLHGNPFVCDCSLYSLLVFWYRRHFSSVMDFKNDYTCRLWS
DSRHSRQVLLLQDSFMNCSDSIINGSFRALGFIHEAQVGERLMVHCDSKTGNA
NTDFIWVGPDNRLLEPDKEMENFYVFHNGSLVIESPRFEDAGVYSCIAMNKQ
RLLNETVDVTINVSNFTVSRSHAHEAFNT
**SEQ ID NO:6 Leucine Zipper Motif**
   LYLSGNFLTQFPMDLYVGRFKL
**SEQ ID NO: 7 Leucine Rich Repeat 1**
   VCPTACICATDIVSCTNKNLSKVPGNLFRLIKR
**SEQ ID NO: 8 Leucine Rich Repeat 2**
   SFAKLNTLILRHNNI
**SEQ ID NO: 9 Leucine Rich Repeat 3**
   TSISTGSFSTTPNLKCLDLSSNKLKTVKNAVFQELKVLEVLLLYN
**SEQ ID NO: 10 Leucine Rich Repeat 4**
   LKVLEVLLLYNNHISYLDPSAFGG
**SEQ ID NO: 11 Leucine Rich Repeat 5**
   LSQLQKLYLSGNFLTQFPMDLYVG
**SEQ ID NO: 12 Leucine Rich Repeat 6**
   LAELMFLDVSYNRIPSMPMHHINLV
**SEQ ID NO: 13 Immunoglobulin (Ig)- domain** 
FRALGFIHEAQVGERLMVHCDSKTGNANTDFIWVGPDNRLLEPDKEMENFY
VFHNGSLVIESPRFEDAGVYSCIAMNKQRLLNETVDVTIN
**SEQ ID NO: 14 PCR Primer 1**
   5'-ATG TCG TTA CGT GTA CAC ACT CTG - 3'
**SEQ ID NO: 15 PCR Primer 2**
   5'-TTA AGT GGA CGC CAC AAA AGG TGT- 3'
**SEQ ID NO:16 S30-21616/DEGA Gene -specific Reverse Primer**
   5'-AAC TCA GGT CCA GTC TCT TAA TCA G- 3'
**SEQ ID NO:17 Forward primer**
   5'- ATG TCG TTA CGT GTA CAC ACT CTG-3'
**SEQ ID NO:18 Reverse Primer**
   5'- CAA AAT GAA AAG ACA GGC AAA CAA ATG-3'
**SEQ ID NO:19 Forward Primer for S30-21616/DEGA-EGFP Fusion Construct**
   5' ATC CTC GAG GCG ACC ATA ATG TCG TTA CGT GTA CAC ACT 3'
**SEQ ID NO:20 Reverse Primer for S30-21616/DEGA-EGFP Fusion Construct**
   5' GAT CAC CGG TGC AGT GGA CGC CAC AAA AGG TGT GTC 3'

## Claims

1. An isolated antisense oligonucleotide, wherein said oligonucleotide inhibits polynucleotide expression of SEQ ID NO:1 or SEQ ID NO:3, wherein the oligonucleotide consists of 20-30 or 12-25 nucleotides of the coding region of SEQ ID NO:1 or SEQ ID NO:3 or the complement thereof.

2. An isolated antisense oligonucleotide of claim 1, where the oligonucleotide is in the form of double stranded DNA, single stranded DNA, RNA or a combination thereof.

3. An isolated antisense oligonucleotide of claim 1 or claim 2, wherein the single stranded DNA is a coding strand or a non-coding strand.

4. An isolated antisense oligonucleotide of any preceding claim, wherin phosphodiester bonds of the oligonucleotide are modified.

5. An *in-vitro* method of inhibiting SEQ ID NO:2 or SEQ ID NO:4 polypeptide expression comprising introducing the oligonucleotide of any one of claims 1 to 4 into a cell, wherein the oligonucleotide inhibits SEQ ID NO:1 or SEQ ID NO:3 polynucleotide expression.

6. The method of claim 5, wherein the oligonucleotide is delivered into a cell by a method selected from the group consisting of: receptor mediated endocytosis; micro-injection; via DNA-protein complexes; and cationic liposomal encapsulation.

7. The method of claim 5, wherein a receptor ligand or cell specific antibodies assist in guiding the oligonucleotide to the cell.

8. Use of an isolated antisense oligonucleotide, wherein said oligonucleotide inhibits SEQ ID NO:1 or SEQ ID NO:3 polynucleotide expression, for the manufacture of a medicament for treating gastric adenocarcinoma.

9. Use according to claim 8, wherein the oligonucleotide is in the form of double stranded DNA, single stranded DNA, RNA or a combination thereof.

10. Use according to claim 8 or 9, wherein the single stranded DNA is a coding strand, a non-coding strand or a combination thereof.

11. Use according to any one of claims 8 to 10, wherein the oligonucleotide comprises a nucleotide sequence of SEQ ID NO:1 or SEQ ID NO:3, a complement thereof, a fragment thereof, or an ORF thereof.

12. Use according to any one of claims 8 to 11, wherein the oligonucleotide comprises 20-30 or 12-25 nucleotides of SEQ ID NO:1 or SEQ ID NO:3.

13. Use according to any one of claims 8 to 12, wherein phosphodiester bonds of the oligonucleotide are modified.

14. An isolated antisense oligonucleotide as defined in any one of claims 8 to 13, for treating gastric adenocarcinoma.

## Patentansprüche

1. Isoliertes Antisense-Oligonucleotid, wobei das Oligonucleotid die Polynucleotidexpression von SEQ ID Nr. 1 oder SEQ ID Nr. 3 hemmt, wobei das Oligonucleotid aus 20-30 oder 12-25 Nucleotiden der Kodierregion von SEQ ID Nr. 1 oder SEQ ID Nr. 3 oder dem Komplement hiervon besteht.

2. Isoliertes Antisense-Oligonucleotid nach Anspruch 1, wobei das Oligonucleotid in Form einer doppelsträngigen DNA, einzelsträngigen DNA, RNA oder einer Kombination hiervon vorliegt.

3. Isoliertes Antisense-Oligonucleotid nach Anspruch 1 oder Anspruch 2, wobei die einzelsträngige DNA ein Kodierstrang oder nicht kodierender Strang ist.

4. Isoliertes Antisense-Oligonucleotid nach einem der vorhergehenden Ansprüche, wobei Phosphodiesterbindungen des Oligonucleotids modifiziert sind.

5. In-vitro Verfahren zur Hemmung einer Polypeptidexpression der SEQ ID Nr. 2 oder SEQ ID Nr. 4 durch Einführen des Oligonucleotids nach einem der Ansprüche 1 bis 4 in eine Zelle, wobei das Oligonucleotid die Polynukleotidexpression der SEQ ID Nr. 1 oder SEQ ID Nr. 3 hemmt.

6. Verfahren nach Anspruch 5, wobei das Oligonucleotid in eine Zelle nach einem Verfahren abgegeben wird, das aus der Gruppe ausgewählt ist, die aus den folgenden besteht: einer Rezeptor-vermittelten Endocytose; einer Mikroinjektion; mittels DNA Proteinkomplexen und durch kationische Liposomeneinkapselung.

7. Verfahren nach Anspruch 5, wobei ein Rezeptorligand oder zellspezifische Antikörper das Führen des Oligonucleotids zur Zelle unterstützen.

8. Verwendung eines isolierten Antisense-Oligonucleotids, wobei das Oligonucleotid eine Polynucleotidexpression der SEQ ID Nr. 1 oder SEQ ID Nr. 3 hemmt, zur Herstellung eines Medikaments zur Behandlung von Adenokarzinom des Magens.

9. Verwendung nach Anspruch 8, wobei das Oligonucleotid in Form einer doppelsträngigen DNA, einzelsträngigen DNA, RNA oder einer Kombination hiervon vorliegt.

10. Verwendung nach Anspruch 8 oder 9, wobei die einzelsträngige DNA ein Kodierstrang, nicht kodierender Strang oder eine Kombination hiervon ist.

11. Verwendung nach einem der Ansprüche 8 bis 10, wobei das Oligonucleotid eine Nucleotidsequenz von SEQ ID Nr. 1 oder SEQ ID Nr. 3, ein Komplement hiervon, ein Fragment hiervon oder einen offenen Leserahmen hiervon umfasst.

12. Verwendung nach einem der Ansprüche 8 bis 11, wobei das Oligonucleotid 20-30 oder 12-25 Nucleotide von SEQ ID Nr. 1 oder SEQ ID Nr. 3 umfasst.

13. Verwendung nach einem der Ansprüche 8 bis 12, wobei Phosphodiesterbindungen des Oligonucleotids modifiziert sind.

14. Isoliertes Antisense-Oligonucleotid gemäß Definition in einem der Ansprüche 8 bis 13 zur Behandlung von Adenokarzinom des Magens.

## Revendications

1. Oligonucléotide antisens isolé, ledit oligonucléotide inhibant l'expression polynucléotidique de la SEQ ID NO: 1 ou de la SEQ ID NO: 3, l'oligonucléotide étant constitué par 20 à 30 ou 12 à 25 nucléotides de la région codante de la SEQ ID NO: 1 ou de la SEQ ID NO: 3 ou de son complément.

2. Oligonucléotide antisens isolé selon la revendication 1, dans lequel l'oligonucléotide se présente sous la forme d'un ADN double brin, d'un ADN simple brin, d'ARN ou d'une de leurs combinaisons.

3. Oligonucléotide antisens isolé selon la revendication 1 ou 2, dans lequel l'ADN simple brin est un brin codant ou un brin non codant.

4. Oligonucléotide antisens isolé selon l'une quelconque des revendications précédentes, dans lequel des liaisons phosphodiester de l'oligonucléotide sont modifiées.

5. Procédé in vitro d'inhibition de l'expression polypeptidique de la SEQ ID NO: 2 ou de la SEQ ID NO: 4, comprenant l'introduction de l'oligonucléotide selon l'une quelconque des revendications 1 à 4, dans une cellule, l'oligonucléotide inhibant l'expression polynucléotidique de la SEQ ID NO: 1 ou de la SEQ ID NO: 3.

6. Procédé selon la revendication 5, dans lequel l'oligonucléotide est délivré dans une cellule via un procédé choisi parmi le groupe constitué par : une endocytose médiée par récepteur, une micro-injection, des complexes d'ADN-protéine et une encapsulation liposomiale cationique.

7. Procédé selon la revendication 5, dans lequel un ligand de récepteur ou des anticorps spécifiques à une cellule apportent leur soutien au guidage de l'oligonucléotide vers la cellule.

8. Utilisation d'un oligonucléotide antisens isolé, ledit oligonucléotide inhibant l'expression polynucléotidique de la SEQ ID NO: 1 ou de la SEQ ID NO: 3, pour la préparation d'un médicament destiné à traiter un adénocarcinome gastrique.

9. Utilisation selon la revendication 8, dans laquelle l'oligonucléotide se présente sous la forme d'un ADN double brin, d'un ADN simple brin, d'ARN ou d'une de leurs combinaisons.

10. Utilisation selon la revendication 8 ou 9, dans laquelle l'ADN simple brin est un brin codant, un brin non codant ou une de leurs combinaisons.

11. Utilisation selon l'une quelconque des revendications 8 à 10, dans laquelle l'oligonucléotide comprend une séquence nucléotidique de la SEQ ID NO: 1 ou de la SEQ ID NO: 3, un de ses compléments, un de ses fragments ou un de ses cadres ouverts de lecture.

12. Utilisation selon l'une quelconque des revendications 8 à 11, dans laquelle l'oligonucléotide comprend de 20 à 30 ou de 12 à 25 nucléotides de la SEQ ID NO: 1 ou de la SEQ ID NO: 3.

13. Utilisation selon l'une quelconque des revendications 8 à 12, dans laquelle des liaisons phosphodiester de l'oligonucléotide sont modifiées.

14. Oligonucléotide antisens isolé tel que défini dans l'une quelconque des revendications 8 à 13, pour traiter l'adénocarcinome gastrique.
